# EUROPEAN PATENT SPECIFICATION

(11) **EP 2 515 676 B1**
(45) Date of publication and mention of the grant of the patent: **25.01.2017**
(21) Application number: 10788322.5
(22) Date of filing: 07.12.2010
(51) Int. Cl.: A23K 20/174, A23K 20/179, A23K 50/75

(54) **USE OF CANTHAXANTHIN AND/OR 25-OH D3 FOR IMPROVED REPRODUCTIVITY AND PERFORMANCE OF ROOSTERS**
VERWENDUNG VON CANTHAXANTHIN UND/ODER 25-OH D3 FÜR VERBESSERTE REPRODUKTIVITÄT UND LEISTUNG VON HÄHNEN
UTILISATION DE CANTHAXANTHINE ET/OU DE 25-HYDROXY VITAMINE D3 (25-OH D3) POUR LA REPRODUCTIVITÉ ET LA PERFORMANCE AMÉLIORÉES DES COQS

(30) Priority: 22.12.2009 EP 09180460
(43) Date of publication of application: 31.10.2012
(73) Proprietor: DSM IP Assets B.V., 6411 TE Heerlen (NL)
(72) Inventor: HERNANDEZ, Jose-Maria, Lavoisier 3925 - Tortuguitas (AR)
(74) Representative: Schwander, Kuno
(86) International application number: PCT/EP2010/069064
(87) International publication number: WO 2011/076557

(56) References cited:
- WO-A2-2010/057811
- DATABASE BIOSIS [Online] BIOSCIENCES INFORMATION SERVICE, PHILADELPHIA, PA, US; 1982, YANG C-P ET AL: "THE EFFECTS OF CANTHAXANTHIN AND VITAMIN A ON THE REPRODUCTIVE PERFORMANCES OF TSAI-YA ANAS-PLATYRHYNCHOS-VAR-DOMESTICA AND THEIR PROGENYS LIVABILITY", XP002620322, Database accession no. PREV198274086138 & NATIONAL SCIENCE COUNCIL MONTHLY, vol. 10, no. 6, 1982, pages 559-567, ISSN: 0250-1651
- SOARES J H ET AL: "25-HYDROXYCHOLECALCIFEROL IN POULTRY NUTRITION", POULTRY SCIENCE, CHAMPAIGN, IL, US, vol. 74, no. 12, 1 January 1995 (1995-01-01), pages 1919-1934, XP009074300, ISSN: 0032-5791
- WILSON H R: "Effects of maternal nutrition on hatchability", 1997, POULTRY SCIENCE, VOL. 76, NR. 1, PAGE(S) 134-143, XP002620323, ISSN: 0032-5791 page 136
- ROBERT F ET AL: "Effects of canthaxanthin supplementation in the ROSS breeder diet on oxidative stress of chicks", 16TH EUROPEAN SYMPOSIUM ON POULTRY NUTRITION : PROCEEDINGS, 26-30 AUGUST, 2007, STRASBOURG, FRANCE, [S.L.] : O.V, [Online] 26 August 2007 (2007-08-26), pages 731-734, XP002565950, Retrieved from the Internet: URL:http://www.cabi.org/animalscience/Uplo ads/File/AnimalScience/addition alFiles/WPSAStrasbourgAug2007/64.pdf> [retrieved on 2010-01-28]
- ATENCIO A ET AL: "TWENTY-FIVE HYDROXYCHOLECALCIFEROL AS A CHOLECALCIFEROL SUBSTITUTE IN BROILER BREEDER HEN DIETS AND ITS EFFECT ON THE PERFORMANCE AND GENERAL HEALTH OF THE PROGENY", POULTRY SCIENCE, CHAMPAIGN, IL, US, vol. 84, no. 8, 1 August 2005 (2005-08-01) , pages 1277-1285, XP009074311, ISSN: 0032-5791
- SOARES J H ET AL: "THE EFFECTIVENESS OF THE VITAMIN D ANALOG 1 .ALPHA.-OH-D3 IN PROMOTING FERTILITY AND HATCHABILITY IN THE LAYING HEN", POULTRY SCIENCE, CHAMPAIGN, IL, US, vol. 58, no. 4, 1 January 1979 (1979-01-01), pages 1004-1006, XP009074301, ISSN: 0032-5791

## Description

### FIELD OF THE INVENTION

The present invention relates to the use of canthaxanthin and 25-hydroxy vitamin D3 (25-OH D3), for improved reproduction and performance of roosters.

### BACKGROUND

To maximize the reproduction rate and performance of roosters, optimal nutritional status of animals is essential.

In accordance with the present invention it has been found that problems in chicken reproduction can be eliminated or substantially ameliorated by administering to the roosters an effective amount of Canthaxanthin or 25-OH-D3, optionally a combination of both nutrients.

Tritsch et al. (US 2003/0170324) disclose a feed premix composition of at least 25-OH D3 in an amount between 5% and 50% (wt/wt) dissolved in oil and an antioxidant, an agent encapsulating droplets of 25-OH D3 and oil, and a nutritional additive (e.g., Vitamin D3). The premix may be added to poultry, swine, canine, or feline food. This composition stabilizes 25-OH D3 against oxidation.

Simoes-Nunes et al. (US 2005/0064018) discloses adding a combination of 25-OH Vitamin D3 and Vitamin D3 to animal feed. In particular, about 10 µg/kg to about 100µg/kg of 25-OH Vitamin D3 and about 200 IU/kg to about 4,000 IU/kg of Vitamin D3 are added to swine feed. This addition improves the pig's bone strength.

Stark et al. (US 5,695,794) disclose adding a combination of 25-OH Vitamin D3 and Vitamin D3 to poultry feed to ameliorate the effects of tibial dyschondroplasia.

Borenstein et al US 5,043,170 discloses the combination of Vitamin D3 and either 1-alpha-hydroxycholecalciferol or 1 alpha, 25-dihydroxycholecalciferol to improve egg strength and leg strength in laying hens and older hens.

Fleshner-Barak (WO 03/007916) discloses administration of bisphosphonate compound and natural vitamin D derivative such as 1,25-dihydroxyvitamin D3 or 24,25-dihydroxyvitamin D3, or 25-OH vitamin D3.

Daifotis et al. (WO 03/086415) disclose inhibiting bone resorption by a combination of at least one bisphosphonate compound and from about 100 IU to about 60,000 IU of a no activated metabolite of vitamin D2 and/or vitamin D3.

The aforementioned documents did not teach or suggest that the use of canthaxanthin and 25-OH D3 or a combination thereof would be surprisingly beneficial to improve reproduction and performance of roosters.

### DETAILED DESCRIPTION OF THE INVENTION

As used throughout the specification and claims, the following definitions apply:
"25-OH D3" refers specifically to 25-hydroxy vitamin D3.

"Rooster", also called a cock or chanticleer, is a male chicken which is meant to include turkeys and ducks.

Canthaxanthin and 25-OH D3 may be obtained from any source, and a composition thereof may be prepared using convenient technology.

The poultry feed is provided which comprises from 10 µg/kg to 100 µg/kg of 25-OH D3 and from 2 to 100 ppm canthaxanthin, preferably 2 to 10 ppm.

Canthaxanthin and 25-hydroxy vitamin D3 are suitably administered together with the food. The term food as used herein comprises both solid and liquid food as well as drinking fluids such as drinking water. Particularly, inventive ingredients can be added as a formulated powder to a premix containing other minerals, vitamins, amino acids and trace elements which is added to regular animal food and thorough mixing to achieve even distribution therein.

In the manufacture of poultry feed in accordance with the invention from 2 ppm to 100 ppm, preferably 2 - 10 ppm of canthaxanthin and from 10µ/kg to about
100µg/kg of 25-hydroxy vitamin D3 are added to regular poultry food. Alternatively, a food premix may be prepared on the basis of regular food components by adding these active ingredients to such food components in higher concentration.

According to the present invention the canthaxanthin compound is available under the Trademark ROVIMIX® Hy-D® 1.25 % and canthaxanthin under the Trademark CAROPHYLL®Red.

According to the present invention it is further advantageous if the composition also contains one or more of the following ingredients: Vitamin A, Vitamin E, Biotin, copper (e.g. as CuSO₄), zinc (e.g. as ZnSO₄), cobalt (e.g. as CoSO₄), selenium (e.g. as Na₂SeO₃), iodine (e.g. as KI), manganese (e.g. as MnSO₄) and/or calcium (e.g. as CaSO₄).

The following non-limiting Examples are presented to better illustrate the invention.

### Example 1: Effect of Carophyll Red (Canthaxanthin) on the productive and reproductive performance of roosters

### Facilities and Equipment

The trial was conducted at the experimental laying house, measuring 210m², using 40 cages for male breeders (0.33 x 0.60 x 0.60 m). This open-type house is equipped with side curtains, and metal roofing. Each cage is fitted with a cup drinker and through feeder.

### Animals

Forty pre-selected 40-week old White Plymouth Rock males were used in the trial, and housed individually in cages.

### Management

The period between the 37^{th} and 39^{th} weeks of age was considered the pre-experimental stage, and included the selection of roosters to be used in the trial. The selection was based on phenotypic assessment, response to abdominal massage for ejaculation stimulation, and semen volume of the ejaculate.

The trial was conducted between weeks 40 and 59 of age, and the experimental period was divided in 5 study periods for body weight and feed consumption evaluation, as follows: period I - 40 to 43 weeks of age, period II - 44 to 47 weeks of age, period III - 48 a 51 weeks of age, period IV - 52 to 55 weeks of age, and period V- 56 to 59 weeks of age.

The roosters were individually weighed every 28 days for body weight measurements. The amount of feed supplied and the leftovers were also weighed for feed consumption calculation. The average body weight at start of the trial was 2,936 grams. During the experimental period, the roosters received the treatments described in Table 1, and were fed ***ad libitum,*** with feed being supplied every day during early morning.

The diets fed during the trial were formulated according to the standard LAVIC feeds for roosters, with or without the addition of the tested product, formulated to meet the requirements according to the life stage of the birds. The diets contained only plant products, and were based on corn and soybean meal.

In 15 day-intervals, semen was collected after 1 p.m. using the abdominal massage method. The rooster was held by the legs, and the breast touched a soft surface on the cage. The obtained semen sample was analyzed for motility, morphological changes, and sperm concentration.

For the assessment of motility and vigor, the fresh semen sample was placed on a slide, covered by a glass slip, and analyzed under a light microscope, at 40x magnification. During the evaluation, the sample was maintained on a heated plate at 40°C. Motility was assessed by comparing the percentage (%) of mobile and immobile spermatozoa, and recording of the percentage of mobile sperm. Vigor was assessed according to a 0 to 5 score scale, being score 0 representative of complete sperm immobility, and score 5 indicative of intense, vigorous, and progressive movement, with wave formation.

For the evaluation of sperm morphology and concentration, samples of the ejaculate were diluted in formalin citrate solution in *Eppendorf* tubes. For measuring sperm concentration, a 10 µl semen sample was added to 1ml of formalin citrate solution, and sperm cells were counted in a *Neubauer* counting chamber following a diagonal line, and the result expressed in number of cells per mm³ of semen. For the final analysis, the results were expressed in number of cells /ml.

For the assessment of morphological anomalies of sperm cells, a 10µl semen sample was added to 1ml of formalin citrate solution, and evaluated using a phase contrast microscope, at a 1000x magnification. One hundred (100) cells were evaluated, and the morphological changes were expressed in percentages.

### Treatments

Table 1 describes the experimental treatments.

**TABLE 1. Experimental treatments used in the trial conducted from August to December 2008, with Plymouth Rock White roosters.**

| **Treatments** | **Carophyll Red (ppm)** |
|---|---|
| 1 | 0 |
| 2 | 60 |

### Experimental Design

The experimental design was completely randomized, with two treatments and 20 repetitions each, where each bird was considered a repetition.

### Results

**TABLE 2. Final body weights (grams) of White Plymouth Rock roosters for each trial period, and averages of all periods.**

| **Treatments** | **Week** | | | | | |
|---|---|---|---|---|---|---|
| | 43rd | 47th | 51st | 55th | 59th | Average |
| Control | 2900 | 2953 | 2895 | 2991 | 3082 | 2952 |
| Carophyll Red | 2967 | 3003 | 2984 | 3058 | 3073 | 3008 |
| P | 0.2139 | 0.3794 | 0.1531 | 0.1611 | 0.9956 | 0.2613 |
| CV | 0.74 | 0.80 | 0.83 | 0.63 | 0.98 | 0.68 |

**TABLE 3. Total feed consumption (grams) of White Plymouth Rock roosters for each period and total average feed consumption**

| **Treatments** | **Periods** | | | | | |
|---|---|---|---|---|---|---|
| | I | II | III | IV | V | Average |
| Control | 3005 | 2956 | 2981 b | 3002 b | 3021 | 2998 |
| Carophyll Red | 3071 | 3090 | 3170 a | 3263 a | 3059 | 3132 |
| P | 0.3168 | 0.1100 | 0.0697 | 0.0223 | 0.9004 | 0.1191 |
| CV | 0.97 | 1.00 | 1.24 | 1.29 | 1.64 | 1.02 |

| | | | | | | |
|---|---|---|---|---|---|---|
| a>b (P< 0.1) - Duncan's test | | | | | | |

**TABLE 4. Daily feed consumption (grams/bird/day) of White Plymouth Rock roosters by experimental period**

| **Treatments** | **Periods** | | | | |
|---|---|---|---|---|---|
| | I | II | III | IV | V |
| Control | 107 | 105 | 106 b | 107 b | 108 |
| Carophyll Red | 110 | 110 | 113 a | 116 a | 109 |
| P | 0.3168 | 0.1100 | 0.0697 | 0.0223 | 0.9004 |
| CV | 0.97 | 1.00 | 1.24 | 1.29 | 1.64 |

| | | | | | |
|---|---|---|---|---|---|
| a>b (P< 0.1) - Duncan's test | | | | | |

**TABLE 5. Morphological changes of spermatozoa (%) of roosters in periods I to III (two collections)**

| **Treatments** | **Periods** | | | | | |
|---|---|---|---|---|---|---|
| | I | | II | | III | |
| | 1° | 2° | 1° | 2° | 1° | 2° |
| Control | 25.6 | 28.3 a | 23.7 a | 13.1 | 18.2 a | 24.3 a |
| Carophyll Red | 28.2 | 24.4 b | 19.2 b | 10.3 | 16.1 b | 18.3 b |
| P | 0.4217 | 0.0006 | 0.0006 | 0.4644 | 0.0100 | 0.0001 |
| CV | 6.31 | 3.78 | 5.75 | 5.80 | 4.81 | 6.09 |

| | | | | | | |
|---|---|---|---|---|---|---|
| a>b (P< 0.1) - Duncan's test | | | | | | |

**TABLE 6. Morphological changes of spermatozoa (%) of roosters in periods IV and V (two collections) and averages for all periods**

| **Treatments** | **Periods** | | | | |
|---|---|---|---|---|---|
| | IV | | V | | I-V |
| | 1st | 2nd | 1st | 2nd | Average |
| Control | 19.3 a | 21.4 | 19.4 a | 25.6 | 21.9 a |
| Carophyll Red | 16.1 b | 19.9 | 17.2 b | 24.6 | 19.6 b |
| P | 0.0001 | 0.1499 | 0.0631 | 0.1855 | 0.0001 |
| CV | 4.46 | 4.94 | 7.71 | 3.26 | 1.89 |

| | | | | | |
|---|---|---|---|---|---|
| a>b (P< 0.1) - Duncan's test | | | | | |

**TABLE 7. Sperm motility (%) of the roosters in periods I to III (two collections)**

| **Treatments** | **Periods** | | | | | |
|---|---|---|---|---|---|---|
| | I | | II | | III | |
| | 1st | 2nd | 1st | 2nd | 1st | 2nd |
| Control | 90.25 | 91.00 | 90.00 | 89.00 | 91.75 | 91.25 |
| Carophyll Red | 92.50 | 92.00 | 90.25 | 91.50 | 92.50 | 92.50 |
| P | 0.9110 | 0.3502 | 0.8780 | 0.1251 | 0.4706 | 0.1492 |
| CV | 2.29 | 1.79 | 2.63 | 2.80 | 1.75 | 1.45 |

**TABLE 8. Sperm motility (%) of the roosters in periods IV and V (two collections) and averages for all periods**

| **Treatments** | **Periods** | | | | |
|---|---|---|---|---|---|
| | IV | | V | | I-V |
| | 1st | 2nd | 1st | 2nd | Average |
| Control | 92.50 | 91.00 | 92.50 | 91.50 | 91.40 b |
| Carophyll Red | 93.00 | 91.25 | 92.75 | 93.75 | 92.50 a |
| P | 0.5372 | 0.7954 | 0.7950 | 0.2811 | 0.0119 |
| CV | 1.37 | 1.60 | 1.77 | 3.89 | 0.72 |

| | | | | | |
|---|---|---|---|---|---|
| a>b (P< 0,1) - Duncan's test | | | | | |

**TABLE 9. Sperm vigor score of roosters in periods I to III period (two collections)**

| **Treatments** | **Periods** | | | | | |
|---|---|---|---|---|---|---|
| | I | | II | | III | |
| | 1st | 2nd | 1st | 2nd | 1st | 2nd |
| Control | 4.71 | 4.35 | 4.50 | 4.38 | 4.28 | 4.35 |
| Carophyll Red | 4.65 | 4.47 | 4.57 | 4.65 | 4.61 | 4.61 |
| P | 0.7239 | 0.6313 | 0.7679 | 0.1994 | 0.1900 | 0.1133 |
| CV | 5.26 | 7.92 | 7.26 | 7.04 | 8.59 | 5.53 |

**TABLE 10. Sperm vigor score of roosters in periods IV and V (two collections) and averages for all periods**

| | **Periods** | | | | |
|---|---|---|---|---|---|
| **Treatments** | IV | | V | | I-V |
| | 1st | 2nd | 1st | 2nd | Average |
| Control | 4.32 | 4.24 | 4.38 | 4.37 | 4.38 b |
| Carophyll Red | 4.53 | 4.31 | 4.61 | 4.53 | 4.56 a |
| P | 0.3237 | 0.7158 | 0.2530 | 0.6271 | 0.0312 |
| CV | 8.16 | 7.57 | 7.06 | 9.55 | 2.84 |

| | | | | | |
|---|---|---|---|---|---|
| a>b (P< 0.1) - Duncan's test | | | | | |

**TABLE 11. Sperm concentration (number of cells x10⁸) of roosters for periods I to III (two collections)**

| | **Periods** | | | | | |
|---|---|---|---|---|---|---|
| **Treatments** | I | | II | | III | |
| | 1st | 2nd | 1st | 2nd | 1st | 2nd |
| Control | 4.69 | 4.00 | 4.67 | 3.94 b | 4.61 b | 5.79 |
| Carophyll Red | 4.82 | 4.31 | 5.06 | 5.05 a | 5.02 a | 5.83 |
| P | 0.5950 | 0.2795 | 0.4657 | 0.0001 | 0.0588 | 0.5485 |
| CV | 5.01 | 4.48 | 3.78 | 3.25 | 2.95 | 3.11 |

| | | | | | | |
|---|---|---|---|---|---|---|
| a>b (P< 0.1) - Duncan's test | | | | | | |

**TABLE 12. Sperm concentration (number of cells x10⁸) of roosters for periods IV and V (two collections) and average for all periods**

| | **Periods** | | | | |
|---|---|---|---|---|---|
| **Treatments** | IV | | V | | I-V |
| | 1st | 2nd | 1st | 2nd | Average |
| Control | 3.00 b | 5.99 b | 4.61 b | 2.65 | 4.40 b |
| Carophyll Red | 3.84 a | 6.46 a | 5.30 a | 2.84 | 4.85 a |
| P | 0.0001 | 0.0084 | 0.0056 | 0.1152 | 0.0002 |
| CV | 2.56 | 1.46 | 2.70 | 2.59 | 1.24 |

| | | | | | |
|---|---|---|---|---|---|
| a>b (P< 0.1) - Duncan's test | | | | | |

### Conclusion

The addition of Carophyll Red to the diets resulted in significant improvements in sperm concentration and vigor, and reduced the incidence of morphological changes seen in spermatozoa produced by White Plymouth Rock roosters during the experimental period, from 40 to 59 weeks of age.

### Example 2: Effect of Carophyll Red and 25-OH-D3 on the reproductive performance of roosters

The trial of example 2 was conducted as described for example 1.

### Treatments

Table 13 describes the four (4) treatments used in this trial.

**TABLE 13 Experimental treatments used in the trial conducted from August to December 2008, with Plymouth Rock White roosters.**

| **Treatments** | **Carophyll Red (ppm)** | **HY-D (ppb)** |
|---|---|---|
| 1 | 0 | 0 |
| 2 | 60 | 0 |
| 3 | 0 | 69 |
| 4 | 60 | 69 |

### Results

**TABLE 14. Final body weights (grams) of White Plymouth Rock roosters for each trial period, and averages of all periods.**

| **Treatments** | **Week of Age** | | | | | |
|---|---|---|---|---|---|---|
| | 43 | 47 | 51 | 55 | 59 | Average |
| Control | 2900 | 2953 | 2895 ab | 2991 ab | 3082 | 2952 |
| Carophyll | 2967 | 3003 | 2984 a | 3058 a | 3073 | 3008 |
| HyD | 2850 | 2870 | 2849 b | 2946 b | 3040 | 2911 |
| Carophyll+HyD | 2933 | 2948 | 3018 a | 3079 a | 3119 | 3012 |
| P | 0.2148 | 0.2497 | 0.0310 | 0.0517 | 0.7460 | 0.2136 |
| CV | 0.79 | 0.93 | 0.85 | 0.69 | 0.96 | 0.75 |

| | | | | | | |
|---|---|---|---|---|---|---|
| a>b (P< 0.1) - Duncan's test | | | | | | |

**TABLE 15. Total feed consumption (grams) of White Plymouth Rock roosters for each period and total average feed consumption**

| **Treatments** | **Periods** | | | | | |
|---|---|---|---|---|---|---|
| | I | II | III | IV | V | Average |
| Control | 3005 | 2956 ab | 2981 b | 3002 b | 3021 | 2998 ab |
| Carophyll | 3071 | 3090 a | 3170 ab | 3263 a | 3059 | 3132 ab |
| HyD | 2937 | 2884 b | 2988 b | 3058 ab | 3009 | 2975 b |
| Carophyll+HyD | 3103 | 3062 a | 3229 a | 3213 ab | 3148 | 3151 a |
| P | 0.1399 | 0.0659 | 0.0324 | 0.0519 | 0.6309 | 0.0704 |
| CV | 1.03 | 1.11 | 1.28 | 1.30 | 1.62 | 1.03 |

| | | | | | | |
|---|---|---|---|---|---|---|
| a>b (P< 0.1) - Duncan's test | | | | | | |

**TABLE 16. Daily feed consumption (grams/bird/day) of White Plymouth Rock roosters by experimental period**

| **Treatments** | **Periods** | | | | |
|---|---|---|---|---|---|
| | I | II | III | IV | V |
| Control | 107 | 105 ab | 106 b | 107 b | 108 |
| Carophyll | 110 | 110 a | 113 ab | 116 a | 109 |
| HyD | 105 | 103 b | 106 b | 109 ab | 107 |
| Carophyll+HyD | 111 | 109 a | 115 a | 114 ab | 112 |
| P | 0.1399 | 0.0659 | 0.0324 | 0.0519 | 0.6309 |
| CV | 1.76 | 1.91 | 2.18 | 2.21 | 2.77 |

| | | | | | |
|---|---|---|---|---|---|
| a>b (P< 0.1) - Duncan's test | | | | | |

**TABLE 17. Morphological changes of spermatozoa (%) of roosters in periods I to III (two collections)**

| **Treatments** | **Periods** | | | | | |
|---|---|---|---|---|---|---|
| | I | | II | | III | |
| | 1st | 2nd | 1st | 2nd | 1st | 2nd |
| Control | 25.6 | 28.3 a | 23.7 a | 13.1 a | 18.2 a | 24.3 a |
| Carophyll | 28.2 | 24.4 b | 19.2 b | 10.3 a | 16.1 b | 18.3 b |
| HyD | 24.9 | 20.5 c | 17.2 c | 12.5 b | 12.0 c | 16.3 c |
| Carophyll+HyD | 25.1 | 19.1 c | 16.4 c | 12.6 a | 13.0 c | 17.5 bc |
| P | 0.3841 | 0.0001 | 0.0001 | 0.0001 | 0.0001 | 0.0001 |
| CV | 4.78 | 3.60 | 6.91 | 5.37 | 5.87 | 5.52 |

| | | | | | | |
|---|---|---|---|---|---|---|
| a>b>c (P<0.1) - Duncan's test | | | | | | |

**TABLE 18. Morphological changes of spermatozoa (%) of roosters in periods IV and V (two collections) and averages for all periods**

| **Treatments** | **Periods** | | | | |
|---|---|---|---|---|---|
| | IV | | V | | I-V |
| | 1st | 2nd | 1st | 2nd | Average |
| Control | 19.3 a | 21.4 a | 19.4 a | 25.6 a | 21.9 a |
| Carophyll | 16.1 b | 19.9 ab | 17.2 b | 24.6 a | 19.6 b |
| HyD | 13.8 c | 18.8 b | 14.3 c | 12.5 b | 16.1 d |
| Carop+HyD | 15.0 bc | 18.7 b | 15.6 bc | 13.7 c | 16.7 c |
| P | 0.0001 | 0.0001 | 0.0001 | 0.0001 | 0.0001 |
| CV | 4.53 | 4.21 | 6.67 | 4.01 | 1.84 |

| | | | | | |
|---|---|---|---|---|---|
| a>b>c>d (P<0.1) - Duncan's test | | | | | |

**TABLE 19. Sperm motility (%) of the roosters in periods I to III (two collections)**

| **Treatments** | **Períodos** | | | | | |
|---|---|---|---|---|---|---|
| | I | | II | | III | |
| | 1st | 2nd | 1st | 2nd | 1st | 2nd |
| Control | 90.25 | 91.00 b | 90.00 | 89.00 b | 91.75 | 91.25 b |
| Carophyll | 92.50 | 92.00 ab | 90.25 | 91.50 ab | 92.50 | 92.50 ab |
| HyD | 90.75 | 93.25 a | 91.50 | 92.25 a | 92.75 | 93.50 a |
| Carophyll+HyD | 89.25 | 93.50 a | 91.25 | 92.00 a | 93.25 | 93.25 a |
| P | 0.2106 | 0.0459 | 0.7272 | 0.0552 | 0.4211 | 0.0587 |
| CV | 2.73 | 1.68 | 2.72 | 2.29 | 1.56 | 1.51 |

| | | | | | | |
|---|---|---|---|---|---|---|
| a>b (P< 0.1) - Duncan's test | | | | | | |

**TABLE 20. Sperm motility (%) of the roosters in periods IV and V (two collections) and averages for all periods**

| **Treatments** | **Periods** | | | | |
|---|---|---|---|---|---|
| | IV | | V | | I-V |
| | 1st | 2nd | 1st | 2nd | Average |
| Control | 92.5 b | 91.00 | 92.50 | 91.50 | 91.40 b |
| Carophyll | 93.00 ab | 91.25 | 92.75 | 93.75 | 92.50 a |
| HyD | 94.25 a | 91.25 | 93.50 | 94.25 | 93.05 a |
| Carophyll+HyD | 93.75 ab | 91.25 | 93.25 | 94.25 | 92.80 a |
| P | 0.0859 | 0.9902 | 0.6979 | 0.2258 | 0.0003 |
| CV | 1.24 | 1.55 | 1.61 | 2.83 | 0.66 |

| | | | | | |
|---|---|---|---|---|---|
| a>b (P< 0.1) - Duncan's test | | | | | |

**TABLE 21. Sperm vigor score of roosters in periods I to III period (two collections)**

| **Treatments** | **Periods** | | | | | |
|---|---|---|---|---|---|---|
| | I | | II | | III | |
| | 1st | 2nd | 1st | 2nd | 1st | 2nd |
| Control | 4.71 | 4.35 | 4.50 | 4.38 | 4.28 | 4.35 |
| Carophyll | 4.65 | 4.47 | 4.57 | 4.65 | 4.61 | 4.61 |
| HyD | 4.40 | 4.75 | 4.59 | 4.65 | 4.68 | 4.40 |
| Carophyll+HyD | 5.00 | 4.00 | 5.00 | 5.00 | 4.00 | 5.00 |
| P | 0.3723 | 0.2390 | 0.8532 | 0.3748 | 0.2624 | 0.2724 |
| CV | 6.46 | 7.00 | 6.72 | 6.52 | 7.94 | 5.58 |

**TABLE 22. Sperm vigor score of roosters in periods IV and V (two collections) and averages for all periods**

| **Treatments** | **Periods** | | | | |
|---|---|---|---|---|---|
| | IV | | V | | I-V |
| | 1st | 2nd | 1st | 2nd | Average |
| Control | 4.32 | 4.24 | 4.38 | 4.37 | 4.38 b |
| Carophyll | 4.53 | 4.31 | 4.61 | 4.53 | 4.56 a |
| HyD | 4.53 | 4.21 | 4.44 | 4.40 | 4.55 a |
| Carophyll+HyD | 5.00 | 4.00 | 5.00 | 4.00 | 4.57 a |
| P | 0.5588 | 0.9403 | 0.5933 | 0.9207 | 0.0827 |
| CV | 7.77 | 7.16 | 7.49 | 9.48 | 2.94 |

| | | | | | |
|---|---|---|---|---|---|
| a>b (P<0.1) - Duncan's test | | | | | |

**TABLE 23. Sperm concentration (number of cells x10⁸) of roosters for periods I to III (two collections)**

| **Treatments** | **Periods** | | | | | |
|---|---|---|---|---|---|---|
| | I | | II | | III | |
| | 1st | 2nd | 1st | 2nd | 1st | 2nd |
| Control | 4.69 | 4.00 c | 4.67 c | 3.94 c | 4.61 c | 5.79 b |
| Carophyll | 4.82 | 4.31 bc | 5.06 bc | 5.05 b | 5.02 b | 5.83 b |
| HyD | 4.43 | 4.73 ab | 5.51 ab | 5.43 b | 5.65 a | 7.52 a |
| Carophyll+HyD | 4.65 | 5.02 a | 6.29 a | 6.19 a | 6.17 a | 7.60 a |
| P | 0.7855 | 0.0034 | 0.0007 | 0.0001 | 0.0001 | 0.0001 |
| CV | 4.37 | 3.71 | 3.56 | 2.56 | 2.57 | 2.46 |

| | | | | | | |
|---|---|---|---|---|---|---|
| a>b>c (P<0.1) - Duncan's test | | | | | | |

**TABLE 24. Sperm concentration (number of cells x10⁸) of roosters for periods IV and V (two collections) and average for all periods**

| **Treatments** | **Periods** | | | | |
|---|---|---|---|---|---|
| | IV | | V | | I-V |
| | 1st | 2nd | 1st | 2nd | Average |
| Control | 3.00 c | 5.99 c | 4.61 d | 2.65 b | 4.40 d |
| Carophyll | 3.84 b | 6.46 b | 5.30 c | 2.84 b | 4.85 c |
| HyD | 4.49 a | 6.42 b | 6.20 b | 3.53 a | 5.40 b |
| Carophyll+HyD | 4.75 a | 7.50 a | 7.22 a | 3.66 a | 5.91 a |
| P | 0.0001 | 0.0001 | 0.0001 | 0.0001 | 0.0001 |
| CV | 2.09 | 1.45 | 2.18 | 3.56 | 1.04 |

| | | | | | |
|---|---|---|---|---|---|
| a>b>c>d (P<0.1) - Duncan's test | | | | | |

### CONCLUSIONS

The addition of HyD or Carophyll Red to the diets resulted in significant improvements in sperm concentration, and reduced the incidence of morphological changes seen in spermatozoa produced by White Plymouth Rock roosters during the experimental period, from 40 to 59 weeks of age.

## Claims

1. The use of canthaxanthin and 25-hydroxy vitamin D3 in a food composition for improving reproductive performance of roosters, wherein the food composition comprises from 10 µg/kg to 100 µg/kg of 25-hydroxy vitamin D3 and from 2 to 100 ppm canthaxanthin,

2. The use according to claim 1, wherein the food composition comprises from 2 ppm to 10 ppm canthaxanthin.

## Patentansprüche

1. Verwendung von Canthaxanthin und 25-Hydroxy-Vitamin D3 in einer Futterzusammensetzung zur Verbesserung der Reproduktionsleistung von Hähnen, wobei die Futterzusammensetzung 10 µg/kg bis 100 µg/kg 25-Hydroxy-Vitamin D3 und 2 bis 100 ppm Canthaxanthin umfasst.

2. Verwendung nach Anspruch 1, wobei die Futterzusammensetzung 2 ppm bis 10 ppm Canthaxanthin umfasst.

## Revendications

1. Utilisation de canthaxanthine et de 25-hydroxyvitamine D3 dans une composition alimentaire pour l'amélioration de la performance de reproduction des coqs, la composition alimentaire comprenant de 10 µg/kg à 100 µg/kg de 25-hydroxyvitamine D3 et de 2 à 100 ppm de canthaxanthine.

2. Utilisation selon la revendication 1, dans laquelle la composition alimentaire comprend de 2 à 10 ppm de canthaxanthine.
